# EUROPÄISCHE PATENTSCHRIFT

(11) **EP 0 817 623 B1**
(45) Veröffentlichungstag und Bekanntmachung des Hinweises auf die Patenterteilung: **12.11.2003**
(21) Anmeldenummer: 96907292.5
(22) Anmeldetag: 22.03.1996
(51) Int. Cl.: A61K 31/00, A61K 31/19, A61K 31/235, A61K 31/606, A61K 31/616, A61P 35/00

(54) **ARZNEISTOFFE ZUR SELEKTIVEN BEKÄMPFUNG VON TUMORGEWEBE**
MEDICAMENTS FOR THE SELECTIVE TREATMENT OF TUMOUR TISSUES
MEDICAMENTS POUR LE TRAITEMENT SELECTIF DES TISSUS AFFECTES PAR DES TUMEURS

(30) Priorität: 30.03.1995 DE 19511623
(43) Veröffentlichungstag der Anmeldung: 14.01.1998
(73) Patentinhaber: Kreutz, Werner, Prof. Dr., 79219 Staufen (DE)
(72) Erfinder: Kreutz, Werner, Prof. Dr., 79219 Staufen (DE)
(74) Vertreter: Keller, Günter, Dr.
(86) Internationale Anmeldenummer: DE9600539
(87) Internationale Veröffentlichungsnummer: WO96030003

(56) Entgegenhaltungen:
- WO-A-80/00791
- WO-A-94/27584
- WO-A-95/24897
- DE-A- 4 407 484
- US-A- 4 724 234
- CHEM.PHARM.BULL., Bd. 32, Nr. 3, 1984, Seiten 1135-1141, XP002011865 S.INAYAMA ET AL.: "Studies on Non-sesquiterpenoid Constituents of Gaillardia pulchella. II. Less Lipophilic Substances, Methyl Caffeate as an Antitumor Catecholic"
- TROPICAL MEDICINE AND INTERNATIONAL HEALTH, Bd. 1, Nr. 3, Juni 1996, Seiten 379-384, XP000579657 P.M.LOISEAU ET AL.: "Plasmodium berghei mouse model: antimalarial activity of new alkaloid salts of thiosemicarbazone and acridine derivatives"
- FITOTERAPIA, Bd. 66, Nr. 5, 1995, Seiten 399-402, XP000579659 J.ULRICHOV ET AL.: "Cytoprotective effect of phenolics from Colchicum on rat hepatocytes"
- EUR.J.PHARMACOL., Bd. 201, Nr. 1, 1991, Seiten 53-58, XP000579658 M.TRAUTMANN ET AL.: "Aspirin-like drugs, ethanol-induced rat gastric injury and mucosal eicosanoid release"
- CANCER RES., Bd. 53, Nr. 4, 15.Februar 1993, Seiten 806-809, XP000579667 M.S.ANDERSON ET AL.: "Enhancement of Merocyanine 540-mediated Phototherapy by Salicylate"
- TUMOR BIOL., Bd. 15, September 1994, Seiten 304-310, XP000577049 T.SEVERIN ET AL.: "pH-Dependent LAK Cell Cytotoxicity" in der Anmeldung erwähnt
- DIALOG (R) FILE 399: CA SEARCH (R), ACCESSION NUMBER 117204558, XP002011866 & CESK. FARM., Bd. 41, Nr. 4-5, 1992, Seiten 127-129,
- S.BUDAVARI ET AL., ED.: "THE MERCK INDEX, 11th Edition" 1989 , MERCK & CO., INC. , RAHWAY, N.J., U.S.A. XP002011867 siehe Seite 78

## Beschreibung

Die Erfindung betrifft neue Arzneimittel, die selektiv Tumorgewebe bekämpfen, während gesundes Gewebe praktisch nicht angegriffen wird. Die neuen Arzneimittel eignen sich daher hervorragend für die Krebstherapie.

Medikamente nach dem Stand der Technik, die bei der Chemotherapie eingesetzt werden, bringen in der Regel nur Teilerfolge, d.h. sie führen zu keiner endgültigen Heilung. Darüber hinaus wirken die im Stand der Technik eingesetzten Substanzen häufig nur bei einer bestimmten Tumorkategorie. Ein weiterer Nachteil der derzeit bekannten Chemotherapeutika sind ihre oft schädlichen Nebenwirkungen, da Chemotherapeutika generell auf proliferierende Gewebe cytostatisch wirken können. Die bekannten Chemotherapeutika sind auch bei der Bekämpfung der Metastasenbildung nicht zufriedenstellend und dies ist einer der Hauptgründe, die bislang einen entscheidenden Erfolg bei der Krebstherapie verhinderten.

Daß Tumorgewebe im extrazellulären Milieu einen abgesenkten mittleren pH-Wert von etwa 6,5 bis 7,0 aufweist und der pH-Wert auf der Krebszellenoberfläche sogar bis 5 absinken kann, während der pH-Wert im Normalgewebe und im Blut etwa 7,2 bis 7,5 beträgt, ist bekannt und wird z.B. in der DE-A 44 07 484 und in Tumor Biol., 1994, 15: 304-310 beschrieben. In diesen Druckschriften wird offenbart, daß durch die Absenkung des pH-Bereiches in Tumorzellen die natürliche Immunabwehr blockiert wird, da die körpereigenen Abwehrzellen mit voller Cytotoxizität nur im leicht basischen Milieu von mehr als 7 auf Krebs-Target-Zellen reagieren. Die DE-A 44 07 484 schlägt daher vor, das saure externe Milieu von Krebszellen auf das normale physiologische pH-Niveau von 7 bis 7,5 zu bringen und dadurch die Krebszellen durch die körpereigene Immunabwehr zu bekämpfen. Hierzu wird das saure externe Milieu von Krebszellen entweder durch künstliche Alkalisierungsmaßnahmen oder durch die Verhinderung des Ansäuerungsprozeßes selbst auf einen physiologischen pH-Wert von 7 bis 7,5 gebracht.

Inayama et al. (Chem. Pharm. Bull., 32, 3, 1982, 1135-1141) und Anderson et al. (Cancer Res., 53, 4, 1993, 806-809) beschreiben beide die verwendung von Dihydroxybenzoesäure als Antikrebsmittel. Diese und die in der DE-A 44 07 484 beschriebenen Arzneimittel stellen zwar einen Fortschritt in der Krebstherapie dar, es wäre jedoch wünschenswert, Arzneimittel zur Verfügung zu haben, die neben der körpereigenen Immunabwehr Tumorzellen selektiv bekämpfen und damit als relativ nebenwirkungsarme Chemotherapeutika verwendet werden können.

Es ist daher eine Aufgabe der vorliegenden Erfindung, Arzneimittel zur Verfügung zu stellen, die eine starke cytotoxische Wirkung weitgehend selektiv auf Tumorgewebe aufweisen. Diese Aufgabe wird durch den Gegenstand der Patentansprüche gelöst.

Während in der DE-A 44 07 484 der Versuch unternommen wurde, den extrazellulären pH-Wert von Tumorgewebe zu erhöhen und damit das Tumorgewebe mittels der körpereigenen Abwehrstoffe zu zerstören, wird vorliegend ein anderer Weg beschritten. Das extrazelluläre Milieu in Tumorgewebe soll hinsichtlich des pH-Wertes gerade nicht verändert werden, sondern der erniedrigte pH-Wert im extrazellulären Tumorgewebe soll als Zielgebiet für Substanzen dienen, die pH-Wert-sensitiv sind, d.h. für Substanzen, die entweder im sauren Milieu protoniert werden und ausschließlich in diesem protonierten Zustand zellzerstörend wirken, oder für Substanzkomplexe, die im sauren Milieu zerfallen und dabei eine Substanz freisetzen, die zellzerstörend wirkt. Solche Substanzen bzw.

Substanzkomplexe werden aufgrund ihrer pH-Sensitivität nur in Krebstumoren und Metastasenbereichen aktiviert und stellen deshalb ein ideales Krebstherapeutikum dar. Auch ist besonders hervorzuheben, daß dieses neue Krebstherapeutikum unabhängig von der speziellen Krebsart generell auf alle Tumortypen wirkt.

Bevorzugt werden in den erfindungsgemäßen Arzneimittel, Verbindungen eingesetzt, die nach ihrer Protonierung zellzerstörend wirken. Die Erfindung wird weiter anhand solcher Verbindungen beschrieben.

Die protonierten Verbindungen können auf verschiedene Art und Weise wirken. So können die Verbindungen nach erfolgter Protonierung reversibel durch die Tumorzellmembran permeieren und auf diese Weise als Protonen-Carrier den Protonengradienten über die Tumorzellmembran abbauen. Der sich einstellende Effekt ist ein Anstieg des extrazellulären pH-Werts und ein Absinken des pH-Wertes im Zellinneren, was bis zum Zelltod der Krebszelle führen kann. Als Nebeneffekt tritt auf, daß solche Substanzen neben ihrer schädigenden Wirkung auf die Krebszelle durch die Erhöhung des pH-Wertes im Außenmilieu die natürliche Immunabwehrreaktion einleiten, wie in der DE-A 44 07 484 beschrieben. Es ist auch möglich Verbindungen einzusetzen, die sich nicht über die Membran von der wäßrigen Außenphase der Membran in die wäßrige Innenphase bewegen, sondern Substanzen, die in die Membran eingebaut werden und einen Protonentransfer durch die Membran ermöglichen. Solche Verbindungen bilden eine definierte Pore für Protonen, d.h. sie erlauben eine freie oder nur schwach eingeschränkte Diffusion von Protonen in Richtung ihres Konzentrationsgradienten.

Nützlich sind ebenfalls Verbindungen, die in protonierter Form Porenbildner sind und die kation-, anion- oder zellsubstratspezifisch wirken. Möglich sind hierbei z.B. Poren für Na⁺, K⁺, Cl⁻, Aminosäuren oder Zucker.

Erfindungsgemäß besonders bevorzugt weisen die Verbindungen aber nach ihrer Protonierung im sauren Milieu toxische Eigenschaften auf und wirken als Zellgifte.

Erfindungsgemäße Arzneimittel enthalten Verbindungen die Molekülteile aufweisen, die bei einem pH-Wert von weniger als 7, vorzugsweise weniger als 6,5 protoniert werden und in diesem protonierten Zustand toxisch für Krebszellen werden. Die Protonierung erfolgt bei einem pH-Wert von mehr als 6. Die Verbindungen weisen bevorzugt ein Dissoziationsgleichgewicht auf, um eine reversible Protonierung bei dem gewünschten pH-Wert sicherzustellen. Die Verbindungen müssen außerdem im Konzentrationsbereich der Wirksamkeit physiologisch verträglich sein, wobei die Toxizitätsgrenze vorzugsweise zwischen 200 mg/kg Körpergewicht/Tag bis 400 mg/kg Körpergewicht/Tag liegt. Die Verbindungen sollen eine geeignete Wasserlöslichkeit besitzen, die vorzugsweise bei mindestens ca. 20 mg/ml besonders bevorzugt bei mindestens 30 mg/ml liegt.

Ein Fachmann kann mit Hilfe der vorstehenden Informationen Verbindungen, die in den erfindungsgemäßen Arzneimittel verwendet werden, unter Verwendung seines allgemeinen Fachwissens und einiger einfacher Versuche, wie sie nachstehend beschrieben werden, auffinden. Die Toxizitätseigenschaften der Verbindungen können experimentell mit der FACScan- und der XTT-ELISA-Technik ermittelt werden. Beide Verfahren sind im Stand der Technik bekannt. Suspendierte Krebszellinien wie z.B. K-562, Raji oder Daudi können mit beiden Techniken, adhärente Krebszellinien lediglich mit der XTT-ELISA-Technik untersucht werden. Für die Untersuchung der Toxizität der Substanzen auf mononukleäre Blutzellen (MNC's) können ebenfalls FACS- und XTT-Techniken eingesetzt werden. Bei der FACS-Technik wird der Anteil toter Zellen durch Propidium-Iodid-Anfärbung, beim XTT-Verfahren durch Umwandlung von XTT durch die Mitochondrien-Dehydrogenase in Formazan nachgewiesen. Die Verfahren sind beispielsweise in Tumor-Biologie 1994, 15, 304 und in dem Biochemiekatalog der Fa. Boehringer Mannheim, Deutschland 0392.C 73.3.1465554 1 CB, Cat. No. 1465 015 beschrieben.

Für die Ermittlung pH-sensitiver toxischer Substanzen läßt man bei verschiedenen vorgegebenen pH-Werten, z.B bei pH 5,5, 6,0, 6,5, 7,0 und 7,5 ausgewählte Substanzen sowohl auf Tumorzellinien als auch auf MNC's einwirken, z.B. 4 Stunden und 24 Stunden, um danach die Zahl der getöteten Krebszellen bzw. MNC's zu messen. Parallel dazu werden Kontrollmessungen ohne Zugabe pH-sensitiver Substanzen durchgeführt. Substanzen sind dann für therapeutische Zwecke geeignet, wenn sie auf Krebszellen gemäß der pH-Charakteristik toxisch wirken und auf MNC's und Erythrozyten im pH-Bereich von pH >7 keine oder nur geringfügige Toxizität aufweisen. Mit diesen in-vitro-Messungen an Zellinien unter definierten experimentellen Bedingungen kann der Fachmann unter Zuhilfenahme seines allgemeinen Fachwissens Verbindungen auffinden, die erfindungsgemäß zur Bekämpfung von Krebszellen geeignet sind.

Zu beachten ist, daß jede Tumorart einen intrinsischen mittleren interzellulären pH-Wert aufweist, der z.B. bei Brusttumoren ca. 6,7 und bei Colontumoren ca. 6,9 beträgt. Um zu vermeiden, daß die Therapie in unmittelbarer Nähe des physiologischen pH-Wertes durchgeführt werden muß und damit unter Umständen Gefährdungen von normalen Zellen und Blutzellen erfolgen, können die pH-Werte im extrazellulären Tumorgewebe um ca. 0,5 pH-Einheiten abgesenkt werden, indem man durch Glucoseverabreichung Acidosen induziert.

Das aromatische System trägt Substituenten, die durch ihren induktiven oder mesomeren Effekt die Dissoziationskonstante der Carboxylgruppe geeignet verändern, so daß eine Protonierung bzw. Deprotonierung im gewünschten pH-Bereich erfolgt.

Die in den erfindungsgemäßen Mitteln eingesetzten Verbindungen weisen die Struktur 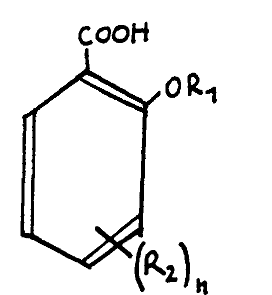 auf, wobei R₁ ein Wasserstoffatom ist, eine Estergruppe oder Ethergruppe vervollständigt und R₂ jeweils unabhängig Aminogruppen, Hydroxylgruppen, Estergruppen, Ethergruppen oder Halogenatome sein kann und n eine ganze Zahl von 1 bis 4 ist.

Die Reste R₁ und R₂ werden so gewählt, daß die Carboxylgruppe bei einem pH-Wert von mehr als 7 deprotoniert vorliegt und in dem gewünschten pH-Bereich, z.B. bei etwa 6,5 protoniert wird. Die Verbindung wird mittels der vorstehend beschriebenen Routineverfahren so ausgewählt, daß sie im deprotonierten Zustand eine wesentlich geringere Cytotoxizität aufweist als im protonierten Zustand.

Als besonders wirkungsvoll haben sich
2,4-Dihydroxybenzoesäure
4-Amino-2-hydroxybenzoesäure
Halogenierte 2-Hydroxybenzoesäure
und deren Derivate
erwiesen.

Als Beispiel wirksamer Derivate werden angeführt:
4-Acetoxy-2-hydroxybenzoesäure
4-Propionsäureester-2-hydroxy-benzoesäure
4-Bernsteinsäureester-2-hydroxybenzoesäure
4-Glutarsäureester-2-hydroxybenzoesäure
4-Acetamino-2-acetoxybenzoesäure
2,4-Diacetoxybenzoesäure
5-Chlor-2-acetoxybenzoesäure
5-Brom-2-acetoxybenzoesäure

Erfindungsgemäß werden 2 oder mehrere Verbindungen im Gemisch eingesetzt, oder nacheinander in derart kurzen Zeitabständen verabreicht, daß sich ihre Wirkungen geeignet ergänzen.

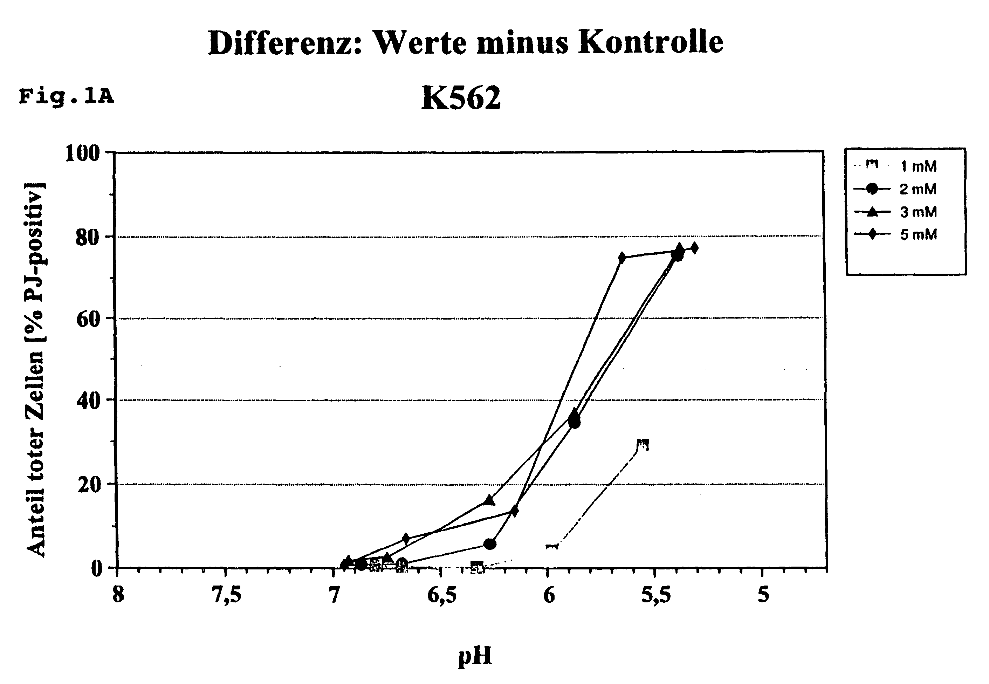

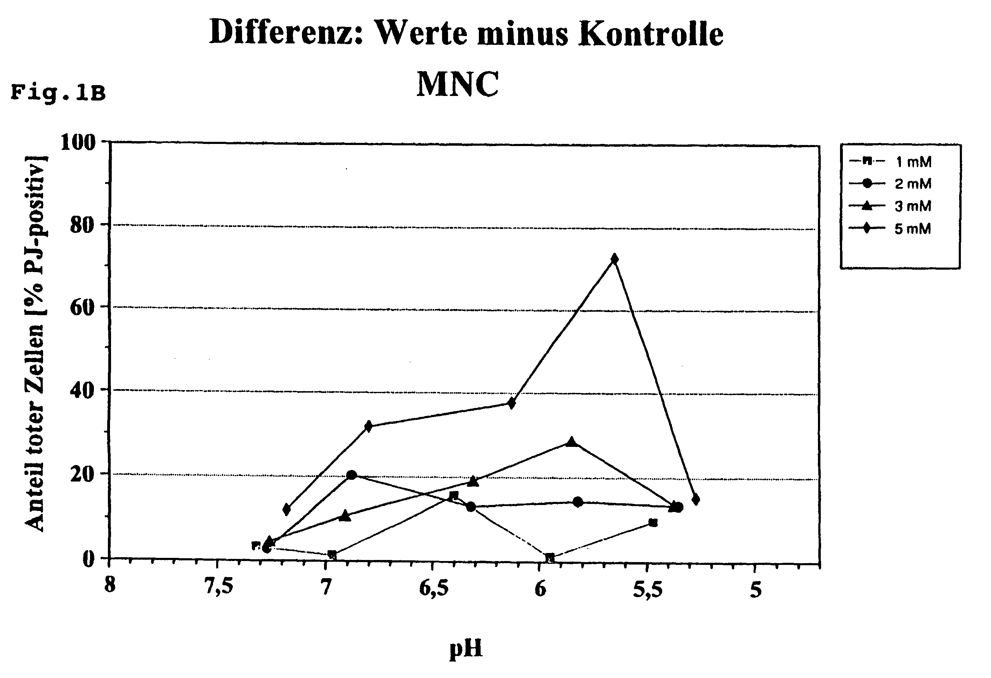

Die Wirksamkeit des 4-Bernsteinsäureesters der 2,4-Dihydroxybenzoesäure wurde überprüft (Vergleichsversuch). Hierbei wurden die vorstehend beschriebenen Methoden der FACScan- und ELISA-Technik verwendet. Figur 1A zeigt die Wirksamkeit des 4-Bernsteinsäureesters der 2,4-Dihydroxybenzoesäure auf K-562-Krebszellen. Es wird deutlich, daß die Toxizitätswirkung bei ca. pH 6,5 einsetzt und bei pH 5,7 ihr Maximum erreicht. In Figur 1B ist die Wirkungsweise der gleichen Verbindung auf mononukleare Blutzellen (MNC's) gezeigt. Bei einem extrazellulären pH-Wert im Bereich von 7,2 bis 7,4, wie er bei normalem Gewebe und bei Blutzellen vorherrscht, kann keine nennenswerte Toxizität festgestellt werden.

Es ist dem Fachmann offensichtlich, daß durch leichte Veränderungen in den Substituenten der vorstehend genannten Verbindungen die pH-Bereiche, bei denen das Dissoziationsgleichgewicht liegt, d.h. bei denen die Protonierung der Verbindungen erfolgt, nach oben oder unten verschoben werden können.

Alle beispielhaft genannten Verbindungen zeigen eine Wasserlöslichkeit von mehr als 30 mg/ml.

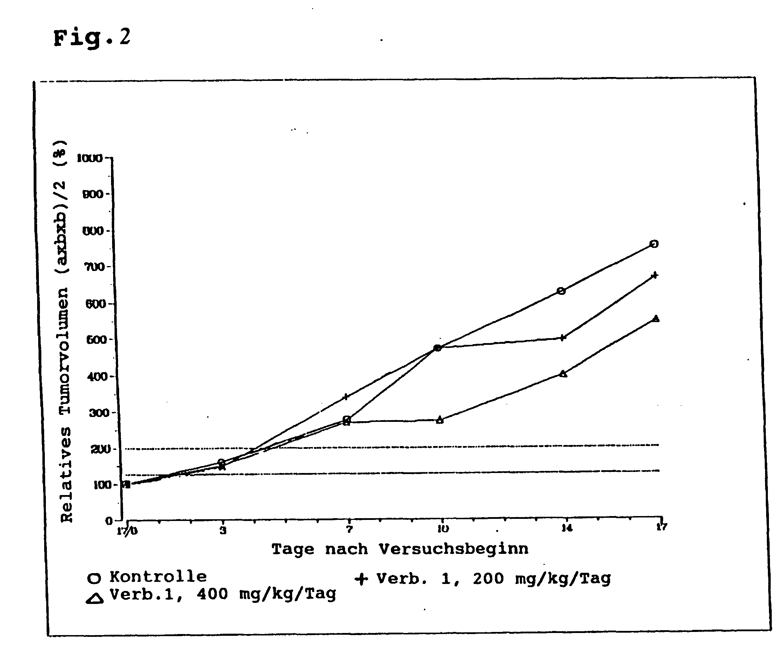

Die vorstehend genannte Verbindung wurde ebenfalls im Tiermodell auf ihre in-vivo-Tauglichkeit überprüft. Als Tiermodell werden Nacktmäuse gewählt, denen verschiedene humane Tumore implantiert werden können. Hierdurch ist es möglich die therapeutische Wirksamkeit der Substanzen unter definierten reproduzierbaren Bedingungen zu überprüfen. In Fig. 2 wird ein derartiger Tierversuch dokumentiert. Es wird ein Routineverfahren, wie es im Stand der Technik üblich und nachstehend skizziert ist, angewandt.

Den Nacktmäusen wird ein kleinzelliger Lungentumor (LXFS 650) implantiert, dessen extrazellulärer pH-Wert von etwa 6,9 nahe dem physiologischen pH-Wert von 7,2-7,3 liegt. Als Therapie-Substanz wird wie bei Fig. 1 2,4-Dihydroxybenzoesäure (Substanz (1)) eingesetzt. 3-4 Wochen nachdem der Tumor eingepflanzt wurde, werden 15 Mäuse, die Tumore von 5-7mm Durchmesser tragen, in 3 Gruppen eingeteilt: die erste 5-er Gruppe wird als Kontrollgruppe ohne Therapie geführt, bei der zweiten 5-er Gruppe wird die Substanz (1) in einer Konzentration von 200 mg/kg/Tag und bei der dritten 5-er Gruppe mit einer Konzentration von 400 mg/kg/Tag in die Schwanzvene injiziert. Im vorliegenden Versuch wurden an 4 aufeinanderfolgenden Tagen 4 Injektionen in die Schwanzvene durchgeführt. Die dadurch gesetzten Venenschäden ließen weitere Injektionen nicht zu. Am 7., 10., 14., und 17. Tag wurden die Tumorvolumen bestimmt. Aus Fig. 2 geht hervor, daß bei einer Konzentration von 400 mg/kg/Tag nach dem 7. Tag ein Wachstumsstillstand erreicht wird bis zum 10. Tag, danach beginnt der Tumor wieder zu wachsen, da die Therapie nicht weiter fortgesetzt wurde. Bei 200 mg/kg/Tag setzt eine schwache Wirkung erst am 10. Tag ein.

Die erfindungsgemäßen Arzneimittel können auf an sich bekannte Art und Weise als Arzneimittel für Säuger, bevorzugt Menschen, formuliert sein. In den erfindungsgemäßen Arzneimitteln liegen die Verbindungen im Gemisch mit einem pharmazeutischen organischen oder anorganischen Träger, der für enterale oder parenterale Verabreichungen geeignet ist, vor. Arzneimittel für die orale Verabreichung, wie Tabletten, Kapseln, Pulver oder eine flüssige Form, wie als Suspension, in Lösung, als Emulsion oder als Sirup sind besonders bevorzugt.

Bei der Formulierung als Tabletten werden übliche Arzneimittelträger wie Natriumcitrat, Lactose, microkristalline Cellulose und Stärke, Schmiermittel wie wasserfreie Kieselsäure, hydriertes Castoröl, Magnesiumstearat, Natriumlaurylsulfat und Talk, sowie Bindemittel wie Stärkepaste, Glucose, Lactose, Gummi-Arabicum Mannit, Magnesiumtrisilicat und Talk verwendet. Wenn es sich bei den erfindungsgemäßen Arzneimittel um Flüßigkeiten handelt, können übliche flüssige Träger verwendet werden.

Bevorzugt ist ebenfalls eine Formulierung für Injektionen und Infusionen, wie es auf dem Fachgebiet bekannt und in einschlägigen Standardwerken beschrieben ist.

Die Dosierungsform der erfindungsgemäßen Arzneimittel hängt sehr stark von der speziellen Verbindung ab und kann von einem Fachmann aufgrund des Zustandes des zu behandelnden Patienten, der Schwere und Art der zu behandelnden Krankheit, sowie möglicher Nebenwirkungen der verabreichten Substanz, bestimmt werden.

## Patentansprüche

1. Arzneimittel enthaltend zumindest zwei verschiedene Verbindungen ausgewählt aus Verbindungen der Formel 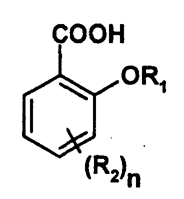 wobei R₁ ein Wasserstoffatom ist, eine Estergruppe oder Ethergruppe vervollständigt und R₂ jeweils unabhängig Aminogruppen, Hydroxylgruppen, Estergruppen, Ethergruppen oder Halogenatome sein kann und n eine ganze Zahl von 1 bis 4 ist, wobei die Verbindungen bei einem pH-Wert von 6 bis 7 protoniert vorliegen und die protonierten Verbindungen auf Zellen stärker zerstörend wirken als die unprotonierten Verbindungen, und einen pharmazeutisch verträglichen inerten Arzneimittelträger.

2. Arzneimittel nach Anspruch 1, bei dem die zumindest zwei verschiedenen Verbindungen im Gemisch vorliegen.

3. Arzneimittel nach Anspruch 1 oder 2, das ferner Glucose enthält.

4. Arzneimittel nach einem der Ansprüche 1 bis 3 zur Verwendung als AntiTumormittel.

5. Verwendung von zumindest zwei verschiedenen Verbindungen, wie in Anspruch 1 definiert, zur Herstellung eines Arzneimittels zur Tumorbekämpfung.

6. Verfahren zur Herstellung eines Arzneimittels zur Tumorbekämpfung, **dadurch gekennzeichnet, daß** zumindest zwei verschiedene Verbindungen wie in Anspruch 1 definiert mit einem pharmazeutisch verträglichen inerten Arzneimittelträger zusammengebracht werden.

7. Verwendung von zumindest zwei Verbindungen, wie in Anspruch 1 definiert, zur Herstellung eines Medikamentes zur Behandlung von Krebs.

## Claims

1. A pharmaceutical composition comprising at least two different compounds selected from compounds of the formula 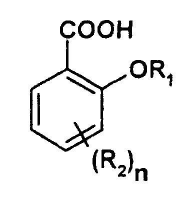 wherein R₁ is a hydrogen atom or completes an ester group or an ether group, and R₂ can in each case be independently amino groups, hydroxyl groups, ester groups, ether groups or halogen atoms, and n is an integer from 1 to 4, wherein the compounds at a pH from 6 to 7 are present in a protonated state and the protonated compounds have a greater destructive effect on cells than the unprotonated compounds, and a pharmaceutically acceptable inert excipient.

2. Pharmaceutical composition as claimed in claim 1, wherein the at least two different compounds are present as a mixture.

3. Pharmaceutical composition as claimed in claim 1 or 2, which furthermore comprises glucose.

4. Pharmaceutical composition as claimed in any of claims 1 to 3 for use as antitumor agent.

5. The use of at least two different compounds as defined in claim 1 for producing a pharmaceutical composition for controlling tumors.

6. A process for producing a pharmaceutical composition for controlling tumors **characterized in that** at least two different compounds as defined in claim 1 are combined with a pharmaceutically acceptable inert excipient.

7. The use of at least two compounds as defined in claim 1 for producing a medicament for the treatment of cancer.

## Revendications

1. Médicament contenant au moins deux composés différents choisis parmi des composés de la formule : 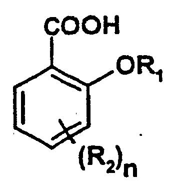 dans laquelle R₁ représente un atome d'hydrogène, ou complète un groupe ester ou un groupe éther et R₂ peut représenter, chaque fois indépendamment, des groupes amino, hydroxyle, ester ou éther ou des atomes d'halogène et n est un nombre entier de 1 à 4, les composés se présentant sous une forme protonée à une valeur de pH de 6 à 7, les composés protonés agissant sur des cellules de manière plus fortement perturbatrice que les composés non protonés, et un support de médicament inerte pharmaceutiquement compatible.

2. Médicament suivant la revendication 1, dans lequel les au moins deux composés différents se présentent en mélange.

3. Médicament suivant l'une des revendications 1 et 2, qui contient en outre du glucose.

4. Médicament suivant l'une des revendications 1 à 3, à utiliser comme produit antitumoral.

5. Utilisation d'au moins deux composés différents, comme défini dans la revendication 1, pour la fabrication d'un médicament destiné à lutter contre les tumeurs.

6. Procédé de préparation d'un médicament destiné à la lutte contre les tumeurs, **caractérisé en ce qu'**au moins deux composés différents, comme défini dans la revendication 1, sont réunis à un support de médicament inerte, pharmaceutiquement compatible.

7. Utilisation d'au moins deux composés, comme défini dans la revendication 1, pour la préparation d'un médicament destiné au traitement du cancer.
